# EUROPEAN PATENT APPLICATION

(11) **EP 3 467 121 A1**
(43) Date of publication of application: **10.04.2019**
(21) Application number: 17802880.9
(22) Date of filing: 25.05.2017
(51) Int. Cl.: C12Q 1/68, C12M 1/00, G01N 33/68

(54) **TARGET BIOLOGICAL MOLECULE IDENTIFICATION METHOD, BEADS FOR TARGET BIOLOGICAL MOLECULE IDENTIFICATION USE, SET OF BEADS, AND TARGET BIOLOGICAL MOLECULE IDENTIFICATION DEVICE**

(30) Priority: 25.05.2016 JP 2016104290
(71) Applicant: Nikon Corporation, Tokyo 108-6290 (JP)
(72) Inventor: TANAKA Shuhei, Tokyo 108-6290 (JP); HAMADA Ryo, Tokyo 108-6290 (JP)
(74) Representative: Hoffmann Eitle
(86) International application number: PCT/JP2017/019530
(87) International publication number: WO 2017/204294

(57) **Abstract**

A method for identifying a target biological molecule includes a step (a) of bringing a sample containing a biological molecule into contact with a plurality of beads on which a ligand capable of binding to one of a plurality of target biological molecules, and a nucleic acid for bead identification which has a specific sequence for each type of the ligand are immobilized; a step (b) of arranging the plurality of beads which have been brought into contact with the sample in each of individual reaction vessels for each bead; a step (c) of adding a reagent necessary for a nucleic acid elongation reaction to the reaction vessel in which the bead is arranged; a step (d) of performing the nucleic acid elongation reaction in the reaction vessel in which the bead is arranged; a step (e) of measuring an amount of protons generated in each of the reaction vessels during the nucleic acid elongation reaction; a step (f) of determining the occurrence or non-occurrence of nucleic acid elongation in each of the reaction vessels based on the amount of protons generated; and a step (g) of performing a nucleic acid elongation reaction in the reaction vessel in which the bead is arranged using the nucleic acid for bead identification as a template, identifying the sequence of the nucleic acid for bead identification for each reaction vessel based on the amount of protons generated in each reaction vessel during the nucleic acid elongation reaction using the nucleic acid for bead identification as a template, and identifying the type of the ligand for each reaction vessel based on the sequence of the nucleic acid for bead identification identified for each reaction vessel.

## Description

### [Technical Field]

The present invention relates to a method for identifying a target biological molecule, a bead for identifying a target biological molecule, a set of beads, and a target biological molecule identification device.

Priority is claimed on Japanese Patent Application No. 2016-104290, filed May 25, 2016, the content of which is incorporated herein by reference.

### [Background Art]

As a method for performing high-throughput nucleic acid analysis, a technique in which, after a primer binds to a bead via a photocleavage site and the primer captures a nucleic acid in a sample, the primer is cleaved by photo-induction, PCR is performed, and a target nucleic acid is amplified to analyze an amplified product is known (refer to Patent Literature 1). However, in the technique disclosed in Patent Literature 1, in a case of performing analysis on the amplified product, it was necessary to recover a bead emulsion to perform sequence analysis and the like.

### [Citation List]

### [Patent Literature]

[Patent Literature 1]
United States Patent Application, Publication No. 2013/0190191

### [Summary of Invention]

An embodiment of the present invention is a method for identifying a target biological molecule, including:
a step (a) of bringing a sample containing a biological molecule into contact with a plurality of beads on which a ligand capable of binding to one of a plurality of target biological molecules, and a nucleic acid for bead identification which has a specific sequence for each type of the ligand are immobilized;
a step (b) of arranging the plurality of beads which have been brought into contact with the sample in each of individual reaction vessels for each bead;
a step (c) of adding a reagent necessary for a nucleic acid elongation reaction to the reaction vessel in which the bead is arranged;
a step (d) of performing the nucleic acid elongation reaction in the reaction vessel in which the bead is arranged;
a step (e) of measuring an amount of protons generated in each of the reaction vessels during the nucleic acid elongation reaction;
a step (f) of determining the occurrence or non-occurrence of nucleic acid elongation in each of the reaction vessels based on the amount of protons generated; and
a step (g) of performing a nucleic acid elongation reaction in the reaction vessel in which the bead is arranged using the nucleic acid for bead identification as a template,
identifying the sequence of the nucleic acid for bead identification for each reaction vessel based on the amount of protons generated in each reaction vessel during the nucleic acid elongation reaction using the nucleic acid for bead identification as a template, and
identifying the type of the ligand for each reaction vessel based on the sequence of the nucleic acid for bead identification identified for each reaction vessel.

Another embodiment of the present invention is a bead for identifying a target biological molecule, on which a ligand capable of binding to a target biological molecule, and a nucleic acid for bead identification which has a specific sequence for each type of the ligand are immobilized.

Still another embodiment of the present invention is a set of beads for identifying a target biological molecule, including a plurality of beads for identifying a target biological molecule, on which a ligand capable of binding to the target biological molecule, and a nucleic acid for bead identification which has a specific sequence for each type of the ligand are immobilized.

Still another embodiment of the present invention is a target biological molecule identification device including: a reaction vessel in which each bead of a plurality of beads on which a ligand capable of binding to one of a plurality of target biological molecules, and a nucleic acid for bead identification which has a specific sequence for each type of the ligand are immobilized, is to be arranged; a detection unit which detects protons generated by a nucleic acid elongation reaction in the reaction vessel in which the bead has been arranged, for each reaction vessel; a nucleic acid elongation determination unit which determines the occurrence or non-occurrence of nucleic acid elongation in the reaction vessel for each reaction vessel based on an amount of protons generated in the reaction vessel, which has been detected by the detection unit; a bead identification unit that identifies the sequence of the nucleic acid for bead identification for each reaction vessel based on the occurrence or non-occurrence of the nucleic acid elongation determined by the nucleic acid elongation determination unit regarding a nucleic acid elongation reaction using the nucleic acid for bead identification as a template; and a target biological molecule identification unit that identifies the type of the ligand for each reaction vessel based on the sequence of the nucleic acid for bead identification identified by the bead identification unit.

### [Brief Description of Drawings]

Fig. 1 is a schematic diagram showing an example of a bead body in a first embodiment.
Fig. 2 is a schematic diagram showing an example of a state in which a target nucleic acid sequence is annealed to a bead body in the first embodiment.
Fig. 3 is a schematic diagram showing an example of arrangement of the bead body in a reaction vessel in the first embodiment.
Fig. 4 is a schematic diagram showing an example of proton release in a nucleic acid elongation reaction.
Fig. 5 is a schematic diagram showing an example of a nucleic acid for bead identification in the present embodiment.
Fig. 6 is a schematic diagram showing examples of the nucleic acid for bead identification in the present embodiment.
Fig. 7 is a diagram showing an example of a configuration of a target nucleic acid sequence identification device in the present embodiment.
Fig. 8 is a diagram showing an example of a configuration of a nucleic acid sequence storage unit in the present embodiment.
Fig. 9 is a schematic diagram showing an example of a sensor in the present embodiment.
Fig. 10 is a schematic diagram showing examples of a bead body in a second embodiment.
Fig. 11 is a diagram showing an example of a configuration of an antigen storage unit in the second embodiment.

### [Description of Embodiments]

A method for identifying a target biological molecule of the present embodiment includes:
a step (a) of bringing a sample containing a biological molecule into contact with a plurality of beads on which a ligand capable of binding to one of a plurality of target biological molecules, and a nucleic acid for bead identification which has a specific sequence for each type of the ligand are immobilized;
a step (b) of arranging the plurality of beads which have been brought into contact with the sample in each of individual reaction vessels for each bead;
a step (c) of adding a reagent necessary for a nucleic acid elongation reaction to the reaction vessel in which the bead is arranged;
a step (d) of performing the nucleic acid elongation reaction in the reaction vessel in which the bead is arranged;
a step (e) of measuring an amount of protons generated in each of the reaction vessels during the nucleic acid elongation reaction;
a step (f) of determining the occurrence or non-occurrence of nucleic acid elongation in each of the reaction vessels based on the amount of protons generated; and
a step (g) of performing a nucleic acid elongation reaction in the reaction vessel in which the bead is arranged using the nucleic acid for bead identification as a template,
identifying the sequence of the nucleic acid for bead identification for each reaction vessel based on the amount of protons generated in each reaction vessel during the nucleic acid elongation reaction using the nucleic acid for bead identification as a template, and
identifying the type of the ligand for each reaction vessel based on the sequence of the nucleic acid for bead identification identified for each reaction vessel.

### <First embodiment>

As an example, the target biological molecule in the method of the present embodiment is a nucleic acid having a desired target nucleic acid sequence. In addition, the ligand capable of binding to the target biological molecule is a primer capable of being annealed to the target nucleic acid sequence (hereinafter referred to as "primer for a target nucleic acid"). In the present embodiment, a case where the target biological molecule is a nucleic acid will be described.

The method of the present embodiment includes:
a step (a1) of bringing a sample containing a nucleic acid into contact with a plurality of beads on which a primer for a target nucleic acid capable of being annealed to one of a plurality of target nucleic acid sequences, and a nucleic acid for bead identification which has a specific sequence for each type of the primer for a target nucleic acid are immobilized;
a step (b1) of arranging the plurality of beads which have been brought into contact with the sample in each of individual reaction vessels for each bead;
a step (c1) of adding a reagent necessary for a nucleic acid elongation reaction to the reaction vessel in which the bead is arranged;
a step (d1) of performing the nucleic acid elongation reaction in the reaction vessel in which the bead is arranged;
a step (e1) of measuring an amount of protons generated in each of the reaction vessels during the nucleic acid elongation reaction;
a step (f1) of determining the occurrence or non-occurrence of nucleic acid elongation in each of the reaction vessels based on the amount of protons generated; and
a step (g1) of performing a nucleic acid elongation reaction in the reaction vessel in which the bead is arranged using the nucleic acid for bead identification as a template,
identifying the sequence of the nucleic acid for bead identification for each reaction vessel based on the amount of protons generated in each reaction vessel during the nucleic acid elongation reaction using the nucleic acid for bead identification as a template, and
identifying the type of the primer for a target nucleic acid for each reaction vessel based on the sequence of the nucleic acid for bead identification identified for each reaction vessel.

Hereinafter, each step will be described.

### [Annealing to primer for target nucleic acid]

The step (a1) is a step of bringing the sample containing the nucleic acid into contact with the plurality of beads on which the primer for a target nucleic acid capable of being annealed to one of the plurality of target nucleic acid sequences, and the nucleic acid for identifying a bead which has a specific sequence for each type of the primer for a target nucleic acid are immobilized.

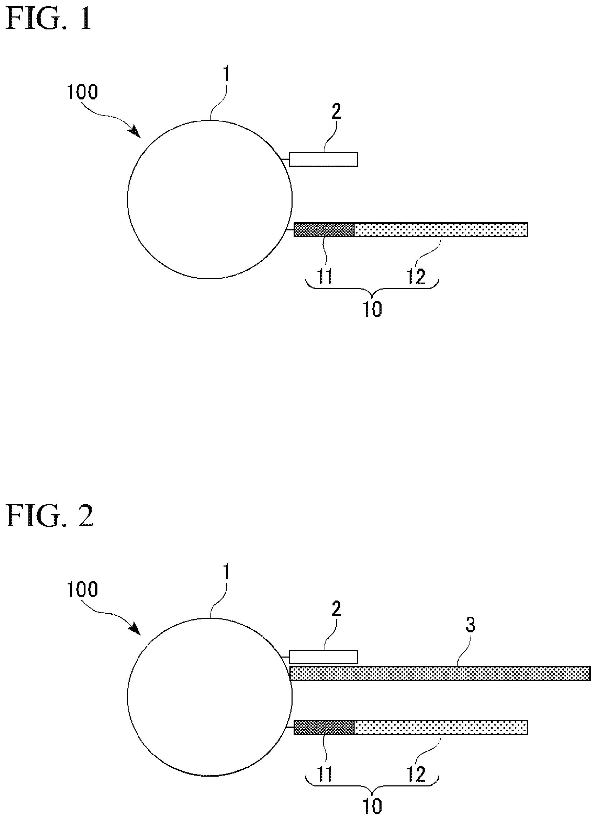

First, the bead used in the present embodiment will be described with reference to Fig. 1. In Fig. 1, a reference numeral 1 denotes the bead, 2 denotes the primer for a target nucleic acid, 10 denotes the nucleic acid for bead identification, 11 denotes a primer annealing region for bead identification, 12 denotes a sequence region for bead identification, and 100 denotes a bead body in which the primer for a target nucleic acid 2 and the nucleic acid for bead identification 10 are immobilized on the bead 1.

In the present embodiment, the bead 1 is used as a carrier for immobilizing the primer for a target nucleic acid 2 and the nucleic acid for bead identification 10. As an example, a shape of the bead 1 is a spherical shape or a shape close thereto. The shape of the bead 1 is not limited thereto. As an example, a size of the bead 1 has an average diameter of 1 to 250 µm. In addition, as an example, the size of the bead 1 has an average diameter of 30 to 80 µm. The size of the bead 1 is not limited thereto.

A material of the bead 1 is not particularly limited, but examples thereof include silicon, titanium dioxide, aluminum oxide, glass, polystyrene, cellulose, polyamide, and the like. In addition, a magnetic bead may be used from the viewpoint of arrangement in the reaction vessel. The bead 1 may be composed of not only one kind of substance but also two or more kinds of substances. Furthermore, the bead 1 may be a bead subjected to surface coating.

As shown in Fig. 1, the primer for a target nucleic acid 2 and the nucleic acid for bead identification 10 are immobilized on the bead 1. The immobilization of these nucleic acids on the bead 1 can be carried out by a known method. For example, the primer for a target nucleic acid 2 and the nucleic acid for bead identification 10 are biotin-modified, and the surface of the bead 1 is coated with avidin, and therefore both nucleic acids can be immobilized to the bead 1 using the avidin-biotin binding. Alternatively, the primer for a target nucleic acid 2 and the nucleic acid for bead identification 10 may be modified with a functional group such as an amino group, a formyl group, or an SH group, and the bead 1 may be subjected to a surface treatment with a silane coupling agent having an amino group, a formyl group, an epoxy group, and the like, and therefore the nucleic acids may be immobilized.

The primer for a target nucleic acid 2 has a sequence capable of being annealed to a desired target nucleic acid sequence. As an example, the primer for a target nucleic acid 2 is a nucleic acid having a sequence complementary to a part of the target nucleic acid sequence. The primer for a target nucleic acid 2 is annealed to the target nucleic acid sequence and induces a nucleic acid elongation reaction using the target nucleic acid sequence as a template. As an example, the primer for a target nucleic acid 2 is DNA. The sequence of the primer for a target nucleic acid 2 can be appropriately selected depending on a desired target nucleic acid sequence. In the present embodiment, the primer for a target nucleic acid 2 each having a different sequence is immobilized on each bead 1.

In the present embodiment, in addition to the primer for a target nucleic acid 2, the nucleic acid for bead identification 10 is immobilized on the bead 1. The nucleic acid for bead identification 10 is for identifying a sequence of the primer for a target nucleic acid 2 immobilized on the bead 1 for each reaction vessel after arranging the bead 1 in the reaction vessel. Depending on the type of the target nucleic acid sequence of the primer for a target nucleic acid 2, the nucleic acid for bead identification 10 each having a different sequence is immobilized. As an example, the nucleic acid for bead identification 10 is DNA.

As an example, the nucleic acid for bead identification 10 has the primer annealing region for bead identification 11 and the sequence region for bead identification 12. As an example, the primer annealing region for bead identification 11 is the same sequence in all beads 1. On the other hands, the sequence region for bead identification 12 each has a different sequence depending on the type of the target nucleic acid sequence of the primer for a target nucleic acid 2. The primer annealing region for bead identification 11 is placed on a 3' side of the nucleic acid for bead identification 10.

In Fig. 1, only one molecule of each of the primer for a target nucleic acid 2 and the nucleic acid for bead identification 10 is immobilized on the bead 1, but the number of molecules of these nucleic acids to be immobilized on the bead 1 is not limited to one molecule. For example, two or more molecules of the primer for a target nucleic acid 2 targeting the same target nucleic acid sequence may be immobilized on the bead 1, and two or more molecules of the nucleic acid for bead identification 10 having the same sequence may be immobilized on the bead 1. As an example, two or more molecules of the primer for a target nucleic acid 2 and the nucleic acid for bead identification 10 are immobilized on the bead 1. The primer for a target nucleic acid 2 and the nucleic acid for bead identification 10 may be immobilized at a density that does not interfere with an annealing and elongation reaction, depending on a surface area of the bead 1. In a case where two or more molecules of the primer for a target nucleic acid 2 and the nucleic acid for bead identification 10 are immobilized on the bead 1, all of the primers for a target nucleic acid 2 immobilized on one bead 1 target the same target nucleic acid sequence. In addition, all of the nucleic acids for bead identification 10 immobilized on one bead 1 have the same sequence.

In the step (a1) of the present embodiment, a bead body 100 in which the primer for a target nucleic acid 2 and the nucleic acid for bead identification 10 are immobilized on the bead 1 is brought into contact with a sample containing a nucleic acid. In the present embodiment, the sample containing the nucleic acid is not particularly limited, and for example, a sample containing a nucleic acid extract or the like of a biological sample or an environmental sample, and the like can be used. As an example, the nucleic acid contained in the sample is DNA. Examples of DNA include cDNA and genomic DNA. The nucleic acid contained in the sample is not limited to DNA, and may be RNA or modified nucleic acid.

Bringing the sample containing the nucleic acid into contact with the bead body 100 can be carried out by, for example, mixing the bead body 100 and the sample containing the nucleic acid. As an example, the mixture of the bead body 100 and the sample containing the nucleic acid is placed in a condition under which the primer for a target nucleic acid 2 can be annealed to the target nucleic acid. Annealing conditions can be appropriately set depending on a length of the primer for a target nucleic acid 2 and the like.

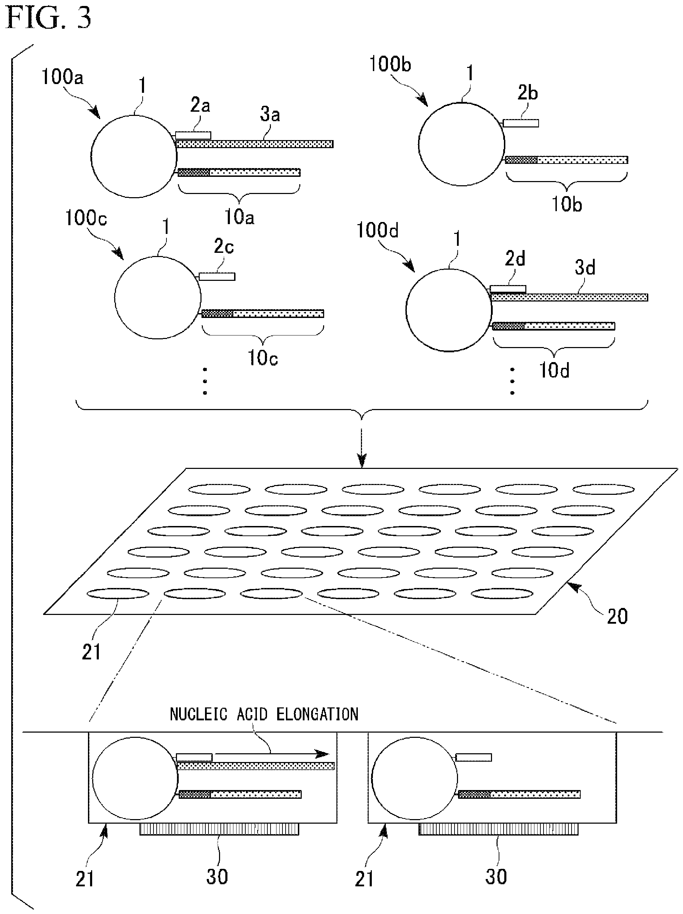

In Fig. 2, a reference numeral 3 denotes the target nucleic acid. The target nucleic acid 3 has a target nucleic acid sequence in the sequence, and the primer for a target nucleic acid 2 can be annealed to at least part of the target nucleic acid sequence. Accordingly, in a case where the target nucleic acid of the primer for a target nucleic acid 2 is contained in the sample, as shown in Fig. 2, the primer for a target nucleic acid 2 is annealed to the target nucleic acid 3, and therefore the target nucleic acid 3 is captured by the bead body 100. On the other hand, in a case where the target nucleic acid of the primer for a target nucleic acid 2 is not contained in the sample, the primer for a target nucleic acid 2 is not annealed to the nucleic acid in the sample. Therefore, in a case where a plurality of bead bodies 100 are brought into contact with the sample, a mixture of a bead body 100 in which the primer for a target nucleic acid 2 is annealed to the target nucleic acid, and a bead body 100 in which the target nucleic acid is not annealed to the primer for a target nucleic acid 2 is formed. In the examples of Fig. 3, in bead bodies 100a and 100d, a primer for a target nucleic acid 2a and a primer for a target nucleic acid 2d are annealed to a target nucleic acid 3a and a target nucleic acid 3d, respectively. On the other hand, in bead bodies 100b and 100c, a primer for a target nucleic acid 2b and a primer for a target nucleic acid 2c are not annealed to the target nucleic acid. The number of bead bodies 100 to be brought into contact with the sample can be appropriately selected according to the number of target nucleic acids to be detected.

### [Arrangement of bead in reaction vessel]

The step (b1) is a step of arranging the bead body 100 which has been brought in contact with the sample containing the nucleic acid in each of the reaction vessels for each bead. The step (b1) of the present embodiment will be described with reference to Fig. 3. In Fig. 3, a reference numeral 20 denotes a substrate for bead arrangement, and 21 denotes a reaction vessel.

In the present embodiment, a material of the substrate for bead arrangement 20 is not particularly limited, but can be glass, silicon, a polymer, or the like. In a case where an elastomeric material is used for the substrate for bead arrangement 20, there is an advantage that a bad influence affecting adhesiveness between all reaction vessels 21 and the substrate for bead arrangement 20, which is caused when particles such as small dust are sandwiched between the reaction vessel 21 and the substrate for bead arrangement 20, is avoided by the local deformation of the elastomer.

On the substrate for bead arrangement 20, a plurality of reaction vessels 21 are arranged. It is required that the number of reaction vessels 21 to be arranged be equal to or more than the number of bead bodies 100 used in the step (a1). As an example, a size of the reaction vessel 21 is slightly larger than the size of the bead 1. As an example, the size of the reaction vessel 21 is about 1 to 2 times the size of the bead 1, and is large enough that one bead 1 can be accommodated. A surface of the substrate for bead arrangement 20 and an inner wall of the reaction vessel 21 may be coated with a blocking agent for preventing nonspecific adsorption of nucleic acids and the like, for example, polyethylene glycol (PEG), 2-methacryloyloxyethyl phosphorylcholine (MPC), and the like. By performing such a coating, the nonspecific adsorption of nucleic acids to the surface of the substrate for bead arrangement 20 and the inner wall of the reaction vessel 21 can be inhibited. In addition, as an example, the inner wall of the reaction vessel 21 has been hydrophilized. When the inner wall has been hydrophilized, in a case where a dispersion liquid of the bead body 100 is added dropwise onto the substrate for bead arrangement 20, an efficiency of filling the reaction vessel 21 with the bead body 100 is improved.

In addition, the reaction vessel 21 has a sensor 30 for detecting a hydrogen concentration. As an example, the sensor 30 is disposed on a bottom surface of the reaction vessel 21. Furthermore, as an example, the sensor 30 is an ion sensitive field effect transistor (ISFET).

In the step (b1) of the present embodiment, as shown in Fig. 3, the plurality of bead bodies 100 which have been brought into contact with the sample containing the nucleic acid are arranged in the reaction vessel 21 one by one. A method for arranging the bead body 100 in the reaction vessel 21 is not particularly limited. For example, a liquid in which the bead body 100 is dispersed is added dropwise onto the substrate for bead arrangement 20, and the bead body 100 may be guided to the reaction vessel 21 by stirring, shaking, centrifugation, and the like. Alternatively, the substrate for bead arrangement 20 may be immersed in a dispersion liquid of the bead body 100, and stirring, shaking, centrifugation, and the like may be used.

In addition, in a case where a magnetic bead is used as the bead 1, it is possible for a magnetic material plate and a magnet to be disposed under a substrate material of the substrate for bead arrangement 20, and for the bead body 100 to be guided to the reaction vessel by the magnetic force of the magnet. In this case, by moving the magnet under the substrate material in a direction parallel to the substrate for bead arrangement 20, the bead body is dispersed, and the efficiency of filling the reaction vessel 21 with the bead body is improved. An intensity of a magnetic field applied to the substrate for bead arrangement 20 by the magnet is not particularly limited, but is 100 to 10000 gauss, for example. In addition, the magnetization of the magnetic plate remains even after removing the magnet, and therefore it is possible to maintain a stable arrangement of the bead body 100. As a material of such a magnetic material, a metal such as nickel, a nickel alloy, iron, and an iron alloy can be suitably used.

### [Addition of reagent necessary for nucleic acid elongation reaction]

The step (c1) is a step of adding a reagent necessary for a nucleic acid elongation reaction to the reaction vessel in which the bead is arranged.

In the present embodiment, examples of the reagent necessary for the nucleic acid elongation reaction include DNA polymerase, a deoxynucleoside triphosphate, an appropriate buffer, and the like. As an example, the DNA polymerase is a thermostable DNA polymerase such as Taq polymerase or a strand-displacement type DNA polymerase. The reagents necessary for the nucleic acid elongation reaction can be appropriately selected according to a method of the nucleic acid elongation reaction carried out in the step (d1) described below. As an example, some of reagents such as DNA polymerase may not be added in this step but may be bound to the inner wall of the reaction vessel 21 in advance.

As an example, in a case of performing a nucleic acid amplification reaction such as a PCR method or an isothermal amplification method as the nucleic acid elongation reaction, a reverse primer can be added in the present step. The reverse primer may be a universal primer common to all the target nucleic acid sequences or may be a primer specific to each target nucleic acid sequence.

The reverse primer can be immobilized on the bead 1 in advance, for example. In addition, in the case where the reverse primer is the universal primer common to all the target nucleic acid sequences, as an example, the universal reverse primer can be immobilized on the inner wall of the reaction vessel 21 in advance. In the case where the reverse primer is immobilized on the bead 1 or the inner wall of the reaction vessel 21, the reverse primer has a photocleavage site on the bead 1 side or the inner wall side of the reaction vessel 21 as an example. The photocleavage site refers to a group having a property of being cleaved upon irradiation with light such as ultraviolet light. Examples of the primer using such a group include PC Linker Phosphoramidite (Glen Research), a composition for photocleavage of nucleic acid which contains fullerene (composition for photocleavage of nucleic acid: Japanese Unexamined Patent Application, First Publication No. 2005-245223), the primer disclosed in Patent Literature 1, and the like. Alternatively, the reverse primer may have a cleavage site for a single-stranded nucleic acid-cleaving enzyme. The cleavage site for the single-stranded nucleic acid-cleaving enzyme refers to a nucleic acid group which can be cleaved by a single-stranded nucleic acid-cleaving enzyme such as deoxyribonuclease and ribonuclease and includes a nucleotide and a derivative thereof. Examples of such a nucleic acid group include deoxyinosine recognized by endonuclease V, and the like. Before starting the nucleic acid amplification reaction, light irradiation or single-stranded nucleic acid-cleaving enzyme treatment is performed to separate the reverse primer from the bead 1, whereby the nucleic acid amplification reaction can be performed efficiently.

### [Nucleic acid elongation reaction]

The step (d1) is a step of performing the nucleic acid elongation reaction in the reaction vessel in which the bead is arranged. The nucleic acid elongation reaction can be carried out by a known method using DNA polymerase and the like. Conditions of the nucleic acid elongation reaction can be appropriately set depending on the method used. For example, the nucleic acid elongation reaction may be carried out while maintaining a temperature in the reaction vessel 21 at about 55 to 70 °C.

The bead bodies 100 are arranged in the reaction vessels 21 one by one. As shown in Fig. 3, in the bead body 100 in which the primer for a target nucleic acid 2 is annealed to the target nucleic acid 3, the nucleic acid elongation reaction occurs using the target nucleic acid 3 as a template. On the other hand, in the bead body 100 in which the primer for a target nucleic acid 2 is not annealed to the target nucleic acid 3, the nucleic acid elongation reaction does not occur.

In the present embodiment, the nucleic acid elongation reaction is not necessarily a nucleic acid amplification reaction. In a case where the density of the primer for a target nucleic acid 2 immobilized on the bead 1 is high, protons sufficient for detection are generated by a single elongation reaction. In the case where the nucleic acid elongation reaction is performed only once, it is not necessary to add the reverse primer to the reaction vessel 21.

Alternatively, in the present embodiment, the nucleic acid amplification reaction can also be performed. As a method of the nucleic acid amplification reaction, a known method can be used. As an example, the PCR method is used for the nucleic acid amplification reaction. In addition, as another example, an isothermal amplification method such as an LAMP method is used. In a case of performing the nucleic acid amplification reaction, it is necessary that the reverse primer be present in the reaction vessel 21 as described above.

### [Detection of proton concentration]

The step (e1) is a step of measuring an amount of protons generated in each of the reaction vessels during the nucleic acid elongation reaction.

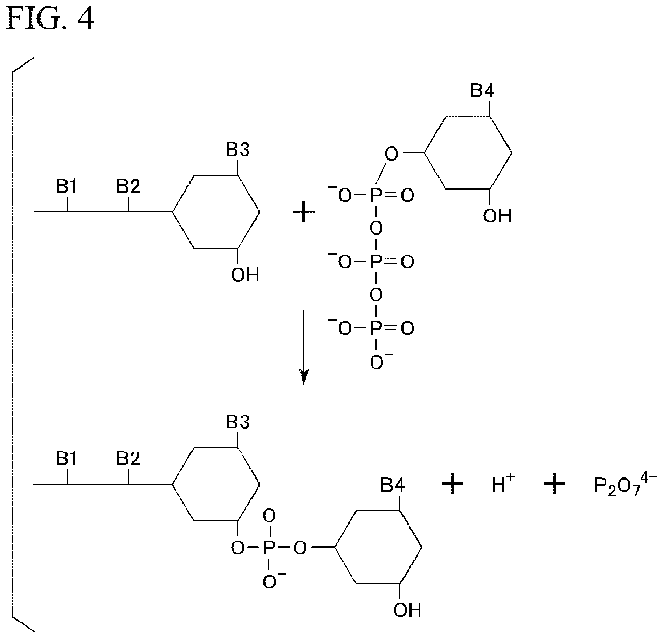

First, the generation of protons in the nucleic acid elongation reaction will be described with reference to Fig. 4. In Fig. 4, B1 to B4 indicate bases. Fig. 4 shows an example in which a deoxynucleoside triphosphate having the base B4 binds to a nucleic acid having a nucleotide sequence of B1-B2-B3 to generate a nucleic acid having a nucleotide sequence of B1-B2-B3-B4. As shown in Fig. 4, when one base is elongated from the nucleic acid having the nucleotide sequence of B1-B2-B3 to generate the nucleic acid having the nucleotide sequence of B1-B2-B3-B4, pyrophosphoric acid and protons of one molecule are released. That is, when one base in the nucleic acid is elongated by the nucleic acid elongation reaction, protons of one molecule are generated. Therefore, by measuring the amount of protons generated in each reaction vessel 21 during the nucleic acid elongation reaction, whether or not nucleic acid elongation has occurred can be determined for each reaction vessel 21. The measured amount of protons generated in the present step is provided for determining the occurrence or non-occurrence of the nucleic acid elongation in the step (f1) to be described later.

The amount of protons generated in the reaction vessel 21 is detected by the sensor 30. As one example, the sensor 30 is the ISFET. Examples of the ISFET that can be used in the present embodiment include a transistor disclosed in PCT International Publication No. WO2008/107014 and the like.

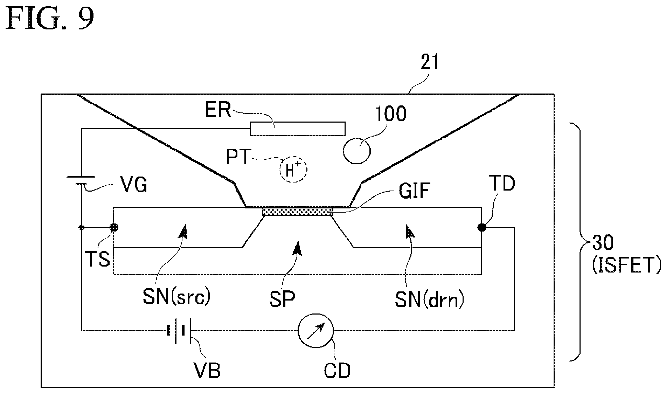

An example of measurement of the amount proton generated in a case where the sensor 30 is the ISFET will be described. Fig. 9 shows a configuration of the sensor 30 provided in each reaction vessel 21. The sensor 30 has a gate insulating film GIF, an N-type semiconductor SN (source src), a source terminal TS, an N-type semiconductor SN (drain drn), a drain terminal TD, a P-type semiconductor SP, a reference electrode ER, a gate power supply VG, a bias power supply VB, and a current detector CD. The configuration of each of these parts is the same as that of the known ISFET, and therefore a description thereof will be omitted. The sensor 30 measures the amount of protons generated by detecting, by the current detector CD, a drain current of a current value corresponding to an interface potential generated between a solution and the gate insulating film GIF.

### [Determination of occurrence or non-occurrence of nucleic acid amplification]

The step (f1) is a step of determining the occurrence or non-occurrence of the nucleic acid elongation in each of the reaction vessels based on the amount of protons generated which is measured in the step (e1). The occurrence or non-occurrence of the nucleic acid elongation in the reaction vessel 21 is determined based on the amount of protons generated in the reaction vessel 21 which is measured by the sensor 30. In a case where the generation of protons is detected in the reaction vessel 21, it is determined that nucleic acid elongation has occurred, and in a case where the generation of protons is not detected in the reaction vessel 21, it is determined that the nucleic acid elongation has not occurred.

In the present embodiment, a change in a proton concentration in each reaction vessel 21 is detected by the sensor 30 disposed for each reaction vessel 21, and the occurrence or non-occurrence of the nucleic acid elongation is determined for each reaction vessel 21. For example, as shown in Fig. 3, in the case where the substrate for bead arrangement 20 has 36 reaction vessels 21, numbers 1 to 36 are assigned to the reaction vessels 21. The sensors 30 are disposed for each reaction vessel 21, and depending on which sensor 30 detected the generation of protons, it is possible to identify in which reaction vessel 21 the nucleic acid elongation reaction has occurred. That is, based on the output of the sensor 30, the number of the reaction vessel 21 in which the nucleic acid elongation reaction has occurred is identified.

There are a plurality of types of bead bodies 100 depending on the types of the sequence of the primer for a target nucleic acid 2. It is not identified which type of the bead body 100 is arranged in which reaction vessel 21 among the plurality of reaction vessels 21. That is, even if the number of the reaction vessel 21 in which the nucleic acid elongation reaction has occurred is identified by the sensor 30, with only this identification, the type of the sequence of the primer for a target nucleic acid 2 in which the nucleic acid elongation reaction has occurred cannot be identified. Hereinafter, a procedure for identifying the primer for a target nucleic acid 2 in each reaction vessel will be described.

### [Identification of primer for target nucleic acid in each reaction vessel]

The step (g1) is a step of performing a nucleic acid elongation reaction in the reaction vessel in which the bead is arranged using the nucleic acid for bead identification as a template, identifying the sequence of the nucleic acid for bead identification for each reaction vessel based on the amount of protons generated in each reaction vessel during the nucleic acid elongation reaction using the nucleic acid for bead identification as a template, and identifying the type of the primer for a target nucleic acid for each reaction vessel based on the sequence of the nucleic acid for bead identification identified for each reaction vessel.

### (Identification of nucleic acid for bead identification)

In the present embodiment, in addition to the primer for a target nucleic acid 2, the nucleic acid for bead identification 10 is immobilized on the bead 1. The type of the sequence of the nucleic acid for bead identification 10 and the type of the target nucleic acid sequence of the primer for a target nucleic acid 2 have a 1:1 correspondence relationship. Therefore, by identifying the type of the sequence of the nucleic acid for bead identification 10, the type of the sequence of the primer for a target nucleic acid 2 can also be identified.

In the present embodiment, as shown in Fig. 1, the nucleic acid for bead identification 10 has the primer annealing region for bead identification 11 and the sequence region for bead identification 12. The primer annealing region for bead identification 11 is a region for annealing of the primer for bead identification in a case of performing the nucleic acid elongation using the sequence region for bead identification 12 as a template. As an example, the sequences of the primer annealing region for bead identification 11 are the same in all of the bead bodies 100. Alternatively, as an example, the sequences of the primer annealing region for bead identification 11 may be changed for every certain number of the bead bodies 100. In this case, in the case of performing the nucleic acid elongation using the sequence region for bead identification to be described later as a template, it is necessary to add all the primers corresponding to the sequences of the used primers annealing region for bead identification 11. The sequence of the primer annealing region for bead identification is not particularly limited. An appropriate primer may be designed and a sequence complementary to the primer may be used.

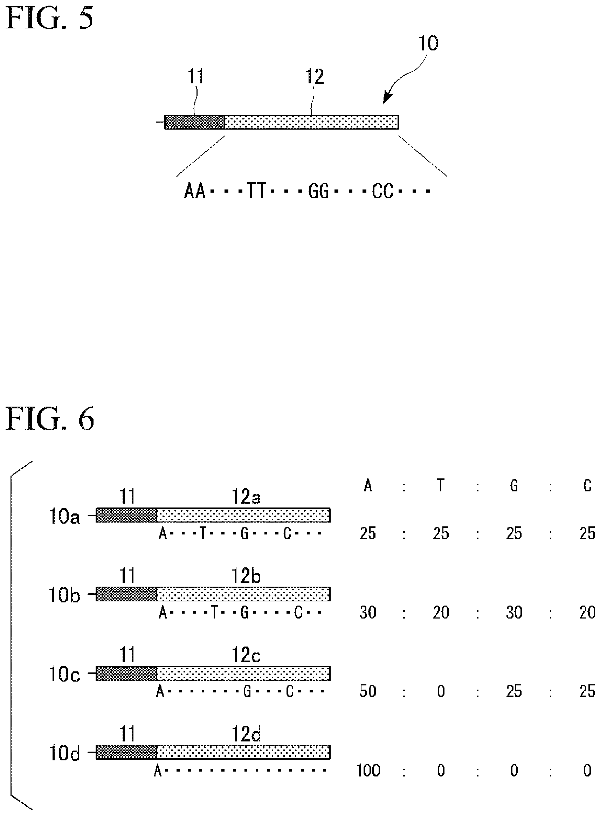

The sequence region for bead identification 12 is a region for identifying the primer for a target nucleic acid 2 immobilized on the bead 1. The sequence of the sequence region for bead identification 12 varies depending on the type of the sequence of the primer for a target nucleic acid 2 to be immobilized on the same bead 1, and has a 1:1 correspondence relationship with the type of a target sequence of the primer for a target nucleic acid 2. As an example, the sequence region for bead identification 12 is any one of a contiguous sequence of adenine (A), a contiguous sequence of guanine (G), a contiguous sequence of cytosine (C), and a contiguous sequence of thymine (T), or a combination of these contiguous sequences. In the example of Fig. 5, the sequence region for bead identification 12 is a sequence in which the contiguous sequences of each of adenine (A), thymine (T), guanine (G), and cytosine (C) are arranged in the above order.

As an example, as shown in Fig. 6, the sequence region for bead identification 12 is a sequence in which the contiguous sequences of each of adenine (A), thymine (T), guanine (G), and cytosine (C) are combined. As an example, the order of the contiguous sequences of each of adenine (A), thymine (T), guanine (G), and cytosine (C) is the same order in the entire sequence region for bead identification 12. In the example of Fig. 6, in sequence regions for bead identification 12a to 12d, contiguous sequences of each of the bases are arranged to be linked in the order of adenine (A), thymine (T), guanine (G), and cytosine (C). Each of the sequence regions for bead identification 12a to 12d has a different number of bases in the contiguous sequences. For example, in Fig. 6, in the sequence region for bead identification 12a, the same number of each of adenine (A), thymine (T), guanine (G), and cytosine (C) are consecutively linked, and in the sequence region for bead identification 12b, the number of adenine (A) and guanine (G) in the contiguous sequence is greater than that of thymine (T) and cytosine (C). The sequence region for bead identification 12 is not required to have all four kinds of the bases. In Fig. 6, in the example of the sequence region for bead identification 12c, thymine (T) is not contained, and in the example of the sequence region for bead identification 12d, the sequence of only adenine (A) is shown.

Next, with reference to Fig. 6 as an example, a method for identifying the nucleic acid for bead identification which are arranged in each reaction vessel will be described. The bead bodies 100 are randomly arranged in each reaction vessel 21, and therefore it cannot be known which nucleic acid for bead identification 10 is arranged in which reaction vessel 21. Accordingly, as an example, the nucleic acid elongation reaction using the sequence region for bead identification 12 as a template is performed, and the sequence of the nucleic acid for bead identification 10 is identified for each reaction vessel based on the amount of protons generated during the nucleic acid elongation reaction. In the example of Fig. 6, the sequence of the nucleic acid for bead identification 10 can be identified by the following procedure.

In the case of the example shown in Fig. 6, when the nucleic acid elongation reaction is carried out with dTTP added to the reaction vessel 21 first, the contiguous sequence of thymidine (T) is synthesized using the contiguous sequence of adenine (A) in the sequence region for bead identification 12 as a template. Accordingly, protons are generated; however, the amount of protons generated varies depending on a length of the contiguous sequence of adenine (A) in the sequence region for bead identification 12. In the example of Fig. 6, the amount of protons generated increases in the order of the sequence regions for bead identification 12a, 12b, 12c, and 12d.

After the generation of protons is no longer detected in any of the reaction vessels 21 during the nucleic acid elongation reaction with the dTTP, next, dATP is added to the reaction vessel 21 to carry out the nucleic acid elongation reaction. In this case, using the contiguous sequence of thymine (T) in the sequence region for bead identification 12 as a template, the contiguous sequence of adenine (A) is synthesized. Accordingly, protons are generated; however, the amount of protons generated varies depending on a length of the contiguous sequence of thymine (T) in the sequence region for bead identification 12. In the example of Fig. 6, the amount of protons generated increases in the order of the sequence regions for bead identification 12b and 12a, and no protons are generated in the sequence regions for bead identification 12c and 12d.

Similarly, the nucleic acid elongation reaction is carried out also with dCTP and dGTP. In the nucleic acid elongation reaction with the dCTP, using the contiguous sequence of guanine (G) in the sequence region for bead identification 12 as a template, the contiguous sequence of cytosine (C) is synthesized. In addition, in the nucleic acid elongation reaction with the dGTP, using the contiguous sequence of cytosine (C) in the sequence region for bead identification 12 as a template, the contiguous sequence of guanine (G) is synthesized. The amount of protons generated during each nucleic acid elongation reaction varies depending on the length of the contiguous sequence of guanine (G) and the contiguous sequence of cytosine (C) in the sequence region for bead identification 12.

In this manner, the nucleic acid elongation reaction is carried out with all four kinds of bases, and the amount of protons generated during the nucleic acid elongation reaction with each base is measured for each reaction vessel. A ratio of the amount of protons generated during the nucleic acid elongation with each base is equal to a ratio of the number of each base contained in the sequence region for bead identification 12 of each nucleic acid for bead identification 10. For example, in a nucleic acid for bead identification 10a of Fig. 6, a ratio of the amount of protons generated during the nucleic acid reaction with each base is dTTP:dATP:dCTP:dGCP = 25:25:25:25. In a nucleic acid for bead identification 10b, a ratio of the amount of protons generated during the nucleic acid reaction with each base is dTTP:dATP:dCTP:dGCP = 30:20:30:20. Similarly, in a nucleic acid for bead identification 10c, a ratio is dTTP:dATP:dCTP:dGCP = 50:0:25:25, and in a nucleic acid for bead identification 10d, a ratio is dTTP:dATP:dCTP:dGCP = 100:0:0:0. Therefore, by calculating the ratio of the amount of protons generated during the nucleic acid elongation reaction with each base for each reaction vessel, the sequence of the sequence region for bead identification 12 of the nucleic acid for bead identification 10 arranged in each reaction vessel can be identified.

Hereinafter, a specific procedure for identifying the sequence of the sequence region for bead identification 12 will be exemplified. As an example, the sequence of the sequence region for bead identification 12 is identified by annealing the primer for bead identification to the primer annealing region for bead identification, and performing the nucleic acid elongation reaction for each of the four kinds of the bases using the sequence region for bead identification 12 as a template.

As an example, the nucleic acid elongation reaction using the sequence region for bead identification 12 as a template is performed after the nucleic acid elongation reaction using the target nucleic acid 3 as a template. In the bead body 100 arranged in the reaction vessel 21 after the step (a1), the primer for a target nucleic acid 2 is in a state of being annealed to the target nucleic acid 3. For this reason, when the nucleic acid elongation reaction is carried out in this state, both nucleic acid elongation reactions of the nucleic acid elongation reaction using the target nucleic acid 3 as a template and nucleic acid elongation reaction using the nucleic acid for bead identification 10 as a template can occur. In this case, there is a concern that protons generated in both nucleic acid elongation reactions may be mixed with each other. Accordingly, as an example, after the steps (d1) and (e1), the target nucleic acid 3 is removed from the reaction vessel 21, or the nucleic acid elongation reaction using the target nucleic acid 3 as a template is stopped. As a result, during performing the nucleic acid elongation reaction using the sequence region for bead identification 12 as a template, mixing of the protons resulting from the nucleic acid elongation reaction using the target nucleic acid 3 as a template can be avoided. As an example, after the completion of the nucleic acid elongation reaction using the target nucleic acid 3 as a template, a temperature inside the reaction vessel 21 is raised to about 90 to 100°C to free the target nucleic acid 3 from the primer for a target nucleic acid 2. Thereafter, the interior of the reaction vessel 21 is washed with an appropriate wash buffer, thereby the target nucleic acid 3 can be removed from the reaction vessel 21. Alternatively, as an example, by adding ddNTP, the nucleic acid elongation reaction using the target nucleic acid 3 as a template can be stopped. Thereafter, the interior of the reaction vessel 21 may be washed with an appropriate wash buffer to remove the ddNTP from the reaction vessel 21.

As an example, the nucleic acid elongation reaction using the sequence region for bead identification 12 as a template can be performed before the nucleic acid elongation reaction using the target nucleic acid 3 as a template. In this case, as an example, the bead body 100 is arranged in each reaction vessel without performing the step (a1), and the step (a1) is performed after the nucleic acid elongation reaction using the target nucleic acid 3 as a template. In this case, as an example, before performing the step (a1), the temperature inside the reaction vessel 21 is raised to about 90 to 100 ° C to free the primer for bead identification from the nucleic acid for bead identification, and by washing the inside of the reaction vessel 21 with an appropriate wash buffer, the primer for bead identification may be removed from the reaction vessel 21.

In a case of performing the nucleic acid elongation reaction using the sequence region for bead identification 12 as a template, the primer for bead identification is added to the reaction vessel 21. The primer for bead identification is annealed to the sequence region for bead identification 12 by setting proper and appropriate annealing conditions according to the sequence of the primer for bead identification. The annealing conditions can be appropriately set according to the sequence of the primer for bead identification.

After the annealing reaction, each kind of dNTPs is added to the reaction vessel 21 to perform the nucleic acid elongation reaction. The order of adding the dNTP is appropriately selected according to the order of the contiguous sequences of each of the bases in the sequence region for bead identification 12. In the example of Fig. 6, the order in the sequence region for bead identification 12 is the order of adenine (A), thymine (T), guanine (G), and cytosine (C), and therefore dTNP, dATP, dCTP, and dGTP are added in this order. The order of the contiguous sequences of each of the bases in the sequence region for bead identification 12 is not limited thereto and can be changed as appropriate. In addition, the contiguous sequences may be repeated twice or more. Furthermore, it is not necessary to use the contiguous sequences of all four kinds of the bases.

During the nucleic acid elongation reaction with each base, the amount of protons generated in the reaction vessel 21 is measured, thereby the amount of protons generated by the nucleic acid elongation reaction at each base is calculated. Then, based on the ratio of the amount of protons generated in the nucleic acid elongation reaction with each base, the sequence of the sequence region for bead identification 12 can be identified for each reaction vessel 21.

In the above-described manner, the sequence of the sequence region for bead identification 12 in each reaction vessel 21 can be identified. As a result, it is possible to identify the type of the nucleic acid for bead identification 10 for each reaction vessel.

### (Identification of primer for target nucleic acid)

In the procedure for identifying the nucleic acid for bead identification 10 described above, the correspondence relationship between the number of the reaction vessel 21 and the type of the sequence of the nucleic acid for bead identification 10 is identified.

The nucleic acid for bead identification 10 and the primer for a target nucleic acid 2 has 1:1 correspondence relationship, and therefore, based on the type of the nucleic acid for bead identification 10 identified above, the sequence of the primer for a target nucleic acid 2 arranged in each reaction vessel 21 is identified.

Then, by checking the occurrence or non-occurrence of the nucleic acid elongation in each reaction vessel 21 determined in the step (f1) and the type of the primer for a target nucleic acid 2 in each reaction vessel 21 identified in this step, the type of the primer for a target nucleic acid 2 in the reaction vessel 21 in which the nucleic acid elongation is determined to be occurred, is identified. That is, a target nucleic acid sequence contained in the sample containing the nucleic acid can be identified.

According to the method of the present embodiment, even if it is not identified that which type of the bead body 100 is arranged in which reaction vessel 21, the primer for a target nucleic acid 2 in which the nucleic acid elongation reaction has occurred can be identified. That is, according to the method of the present embodiment, even if the correspondence relationship between the type of the bead body 100 and the number of the reaction vessel 21 is not identified, the target nucleic acid sequence contained in the sample can be identified.

According to the method of the present embodiment, before arranging the bead 1 in the reaction vessel 21, the sample can be brought into contact with the bead 1 on which the primer for a target nucleic acid 2 is immobilized, and therefore annealing of the primer for a target nucleic acid 2 to the target nucleic acid can be efficiently performed.

According to the method of the present embodiment, it is possible to efficiently detect a plurality of target nucleic acid sequences in the sample containing the nucleic acid.

The method of the present embodiment can be used for detection and identification of pathogenic genes responsible for infectious diseases, detection and identification of genes related to disease such as cancers, expression analysis of disease-related genes, and the like, and is useful for diagnosis of diseases such as infectious diseases and cancers, and monitoring of therapeutic effects.

### <Second embodiment

As an example, a target biological molecule in the method of the present embodiment is a desired protein. In addition, a ligand capable of binding to the target biological molecule is an antibody capable of binding to the protein (hereinafter referred to as "antibody for detecting a target antigen"). In the present embodiment, a case where the target biological molecule is the protein will be described.

The method of the present embodiment includes:
a step (a2) of bringing a sample containing a protein into contact with a plurality of beads on which an antibody for detecting a target antigen which is capable of binding to one of target proteins, and a nucleic acid for bead identification which has a specific sequence for each type of the antibody are immobilized;
a step (b2) of arranging the plurality of beads which have been brought into contact with the sample in each of individual reaction vessels for each bead;
a step (c2) of adding a reagent necessary for a nucleic acid elongation reaction to the reaction vessel in which the bead is arranged;
a step (d2) of performing the nucleic acid elongation reaction in the reaction vessel in which the bead is arranged;
a step (e2) of measuring an amount of protons generated in each of the reaction vessels during the nucleic acid elongation reaction;
a step (f2) of determining the occurrence or non-occurrence of nucleic acid elongation in each of the reaction vessels based on the amount of protons generated; and
a step (g2) of performing a nucleic acid elongation reaction in the reaction vessel in which the bead is arranged using the nucleic acid for bead identification as a template,
identifying the sequence of the nucleic acid for bead identification for each reaction vessel based on the amount of protons generated in each reaction vessel during the nucleic acid elongation reaction using the nucleic acid for bead identification as a template, and
identifying the type of the antibody for detecting a target antigen for each reaction vessel based on the sequence of the nucleic acid for bead identification identified for each reaction vessel.

Hereinafter, each step will be described.

### [Antigen-antibody reaction with target protein]

The step (a2) is a step of bringing the sample containing the protein into contact with the plurality of beads on which the antibody for detecting a target antigen which is capable of binding to one of target proteins, and the nucleic acid for bead identification which has a specific sequence for each type of the antibody for detecting a target antigen are immobilized.

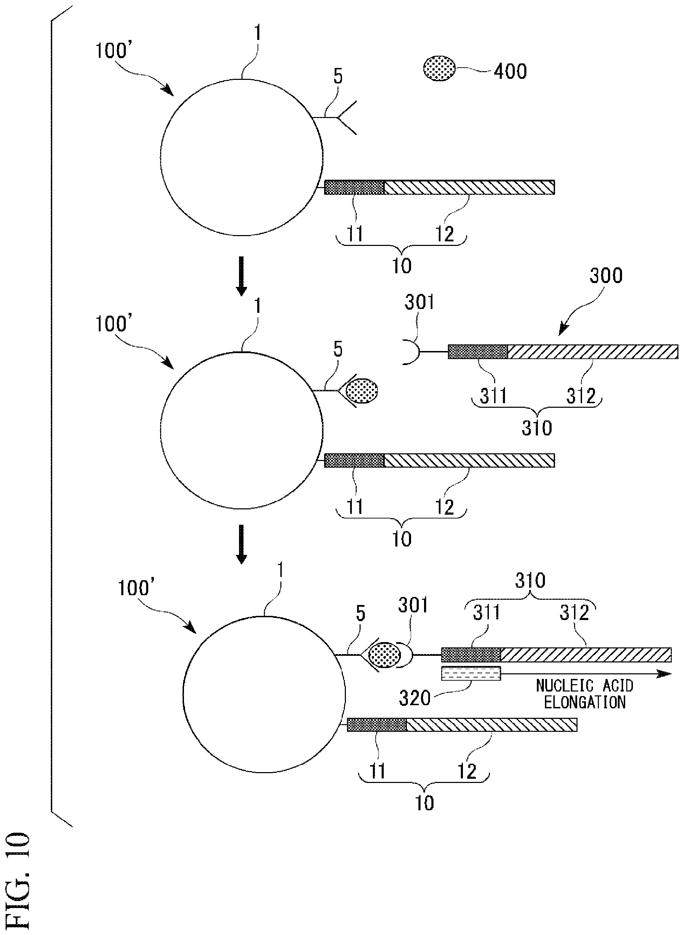

First, the bead used in the present embodiment will be described with reference to Fig. 10. In Fig. 10, a reference numeral 1 denotes the bead, 5 denotes the antibody for detecting a target antigen, 10 denotes the nucleic acid for bead identification, 11 denotes a primer annealing region for bead identification, 12 denotes a sequence region for bead identification, 100' denotes a bead body in which the antibody for detecting a target antigen 5 and the nucleic acid for bead identification 10 are immobilized on the bead 1, and 400 denotes the target protein.

In the present embodiment, instead of the primer for a target nucleic acid 2 of the first embodiment, the antibody for detecting a target antigen 5 is immobilized on the bead 1. The antibody for detecting a target antigen 5 is an antibody capable of specifically binding to a desired target protein. The antibody for detecting a target antigen 5 can be obtained by a known method for obtaining an antibody. The antibody for detecting a target antigen 5 may be an antibody derived from any animal. Antibodies generally used in the technical field such as mouse antibodies, chicken antibodies, and rabbit antibodies can be used. Furthermore, the antibody for detecting a target antigen 5 may be a recombinant antibody such as a chimeric antibody and a humanized antibody. The antibody for detecting a target antigen 5 may be an antibody fragment such as Fab fragment, F(ab')₂ fragment, Fv fragment, and a single chain antibody. In the present specification, the term "antibody" includes antibody fragments thereof in which specificity and binding property to the antigen is maintained.

The immobilization of the antibody for detecting a target antigen 5 on the bead 1 can be carried out by a known method. For example, in the same manner as in the primer for a target nucleic acid 2 of the first embodiment, the immobilization can be carried out by using the avidin-biotin binding. Alternatively, the antibody for detecting a target antigen 5 may be modified with a functional group such as an amino group, a formyl group, or an SH group, and the bead 1 may be subjected to a surface treatment with a silane coupling agent having an amino group, a formyl group, an epoxy group, and the like, and therefore the antibody may be immobilized.

A plurality of molecules of the antibody for detecting a target antigen 5 can be immobilized on the bead 1. As an example, two or more molecules of the antibody for detecting a target antigen 5 are immobilized on the bead 1. The antibody for detecting a target antigen 5 may be immobilized at a density that does not interfere with annealing and elongation reaction, depending on a surface area of the bead 1. In a case where two or more molecules of the antibody for detecting a target antigen 5 are immobilized on the bead 1, all of the antibody for detecting a target antigen 5 immobilized on one bead 1 target the same protein.

A different type of the nucleic acid for bead identification 10 is immobilized on the bead 1 for each kind of the protein targeted by the antibody for detecting a target antigen 5. The nucleic acid for bead identification 10 is the same as in the first embodiment except that the nucleic acid for bead identification 10 corresponds to the antibody for detecting a target antigen 5 instead of the primer for a target nucleic acid 2. The bead 1 is the same as in the first embodiment.

In the step (a2) of the present embodiment, the a bead body 100' in which the antibody for detecting a target antigen 5 and the nucleic acid for bead identification 10 are immobilized on the bead 1 is brought into contact with the sample containing the protein. In the present embodiment, the sample containing the protein is not particularly limited, and for example, a sample containing a protein extract or the like of a biological sample or an environmental sample, and the like can be used. The protein contained in the sample is not limited to a natural protein, and may be a modified protein, a recombinant protein, and the like.

Bringing the sample containing the protein into contact with the bead body 100' can be carried out by, for example, mixing the bead body 100' and the sample containing the protein. As an example, the mixture of the bead body 100 and the sample containing the protein is placed in a condition under which the antibody for detecting a target antigen 5 can bind to the target protein. Binding conditions can be appropriately set according to the type of the antibody for detecting a target antigen 5 and the like.

In Fig. 10, when the bead body 100' is brought into contact with the sample containing the protein, in a case where the sample contains the target protein 400, the antibody for detecting a target antigen 5 binds to the target protein 400. That is, the target protein 400 binds to the antibody for detecting a target antigen 5 and is captured by the bead body 100'. On the other hand, in a case where the target protein 400 is not contained in the sample, no substance binds to the antibody for detecting a target antigen 5. Therefore, in a case where a plurality of bead bodies 100' are brought into contact with the sample, a mixture of the bead body 100' in which the antibody for detecting a target antigen 5 binds to the target protein 400, and the bead body 100' in which the antibody for detecting a target antigen 5 does not bind to the target protein 400 is formed. The number of bead bodies 100 to be brought into contact with the sample can be appropriately selected according to the number of target proteins to be detected.

### [Arrangement of bead in reaction vessel]

The step (b2) of the present embodiment can be carried out in the same manner as the step (b1) of the first embodiment.

### [Addition of reagent necessary for nucleic acid elongation reaction]

The step (c2) is a step of adding a reagent necessary for the nucleic acid elongation reaction to the reaction vessel in which the bead is arranged.

In the step (c2) of the present embodiment, a secondary antibody 301 to which a nucleic acid for signal generation 310 binds is added in addition to the reagent added in the step (c1) of the first embodiment. The secondary antibody 301 is an antibody capable of specifically binding to the target protein 400. The secondary antibody 301 may be the same antibody as the antibody for detecting a target antigen 5 or may be a different antibody. As an example, the secondary antibody 301 is an antibody against the same antigen as the antibody for detecting a target antigen 5 and is an antibody having a different epitope.

As an example, as shown in Fig. 10, the nucleic acid for signal generation 310 is bound to the secondary antibody 301. The binding between the secondary antibody 301 and the nucleic acid for signal generation 310 can be performed by utilizing the avidin-biotin binding, for example.

As an example, as shown in Fig. 10, the nucleic acid for signal generation 310 has a primer annealing region 311 and a sequence for signal generation 312. The primer annealing region 311 is located at a 3' side of the nucleic acid for signal generation 310. As an example, the nucleic acid for signal generation 310 can be the same sequence for all types of secondary antibodies. In addition, as an example, in the nucleic acid for signal generation 310, the primer annealing region 311 is the same sequence for all types of secondary antibodies. The sequence of the nucleic acid for signal generation 310 is not particularly limited and can be appropriately selected. In Fig. 10, a reference numeral 300 denotes a complex probe in which the nucleic acid for signal generation 310 is bound to the secondary antibody 301.

In the present step, as an example, the complex probe 300 is added to the reaction vessel 21 before adding other reagents necessary for the nucleic acid elongation reaction. In this case, as an example, if the bead 1 is a magnetic bead, by immobilizing the bead 1 to the reaction vessel 21, and washing the reaction vessel 21, the complex probes 300 not binding to the target protein 400 can be removed from the reaction vessels 21. The complex probe 300 may be added to the sample or the reaction vessel 21 at any timing as long as addition is performed before the nucleic acid elongation reaction in the step (d2) to be described later.

In the present step, in addition to the complex probe 300, a primer for signal generation 320 is further added. The primer for signal generation 320 is added to the reaction vessel 21 after adding the complex probe 300. As an example, before adding the primer for signal generation 320, the complex probe 300 not binding to the target protein 400 is removed from the reaction vessel 21 by washing. As an example, the addition of the primer for signal generation 320 to the reaction vessel 21 can be performed together with other reagent necessary for the nucleic acid elongation.

The reagent necessary for the nucleic acid elongation reaction other than the above-described reagent is the same as that of the step (c1) of the first embodiment.

### [Nucleic acid elongation reaction]

The step (d2) is a step of performing the nucleic acid elongation reaction in the reaction vessel in which the bead is arranged. The present step can be carried out in the same manner as the step (d1) of the first embodiment. In the present step, as shown in Fig. 10, a nucleic acid elongation reaction using the sequence for signal generation 312 as a template occurs. As in the first embodiment, in a case where the target protein 400 does not bind to the antibody for detecting a target antigen 5, the nucleic acid elongation reaction does not occur.

### [Detection of proton concentration]

The step (e2) is a step of measuring an amount of protons generated in each of the reaction vessels during the nucleic acid elongation reaction. The present step can be carried out in the same manner as the step (e1) of the first embodiment.

### [Determination of occurrence or non-occurrence of nucleic acid amplification]

The step (f2) is a step of determining the occurrence or non-occurrence of the nucleic acid elongation in each of the reaction vessels based on the amount of protons generated which is measured in the step (e2). The present step can be carried out in the same manner as the step (f1) of the first embodiment.

### [Identification of antibody for detecting target antigen in each reaction vessel]

The step (g2) is a step of performing a nucleic acid elongation reaction in the reaction vessel in which the bead is arranged using the nucleic acid for bead identification as a template, identifying the sequence of the nucleic acid for bead identification for each reaction vessel based on the amount of protons generated in each reaction vessel during the nucleic acid elongation reaction using the nucleic acid for bead identification as a template, and identifying the type of the antibody for detecting a target antigen for each reaction vessel based on the sequence of the nucleic acid for bead identification identified for each reaction vessel.

The present step can also be carried out in the same manner as in the first embodiment except that the antibody for detecting a target antigen 5 is used instead of the primer for a target nucleic acid 2 in the first embodiment. That is, as in the first embodiment, the sequence of the nucleic acid for bead identification 10 in each reaction vessel is identified, and based on the sequence of the nucleic acid for bead identification 10, the type of the antibody for detecting a target antigen 5 in each reaction vessel can be identified.

Then, by checking the occurrence or non-occurrence of the nucleic acid elongation in each reaction vessel 21 determined in the step (f2) and the type of the antibody for detecting a target antigen 5 in each reaction vessel 21 identified in this step, the type of the antibody for detecting a target antigen 5 in the reaction vessel 21 in which the nucleic acid elongation is determined to be occurred, is identified. That is, the target protein contained in the sample containing the protein can be identified.

According to the method of the present embodiment, even if it is not identified that which type of the bead body 100' is arranged in which reaction vessel 21, the antibody for detecting a target antigen 5 to which the target protein 400 bound can be identified. That is, according to the method of the present embodiment, even if the correspondence relationship between the type of the bead body 100' and the number of the reaction vessel 21 is not identified, the target protein contained in the sample can be identified.

According to the method of the present embodiment, before arranging the bead 1 in the reaction vessel 21, the sample can be brought into contact with the bead 1 on which the antibody for detecting a target antigen 5 is immobilized, and therefore binding of the antibody for detecting a target antigen 5 to the target protein can be efficiently performed.

According to the method of the present embodiment, it is possible to efficiently detect a plurality of target proteins in the sample containing the protein.

The method of the present embodiment can be used for detection and identification of pathogenic antigens responsible for infectious diseases, detection and identification of genes related to disease such as cancers, expression analysis of disease-related genes, and the like, and is useful for diagnosis of diseases such as infectious diseases and cancers, and monitoring of therapeutic effects.

### <Target biological molecule identification device>

### [Configuration of target nucleic acid sequence identification device]

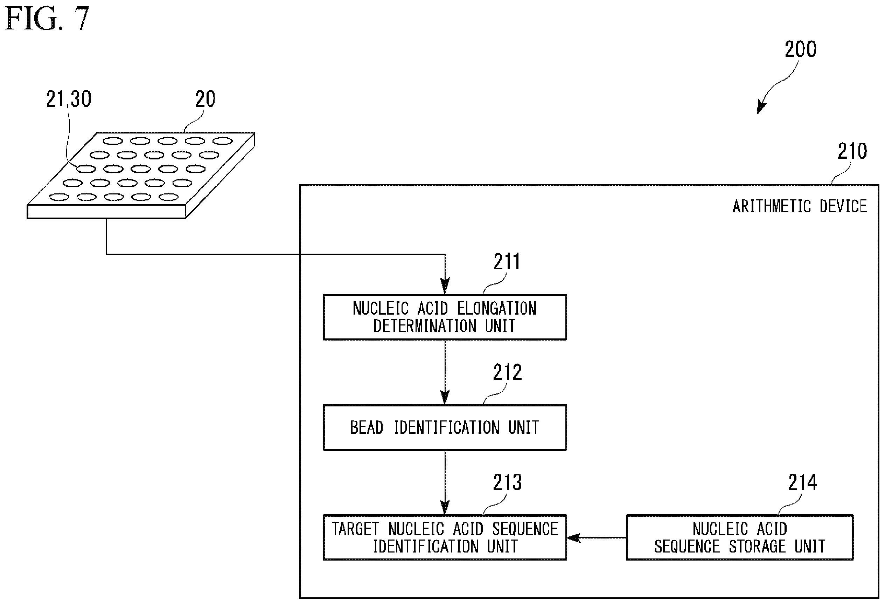

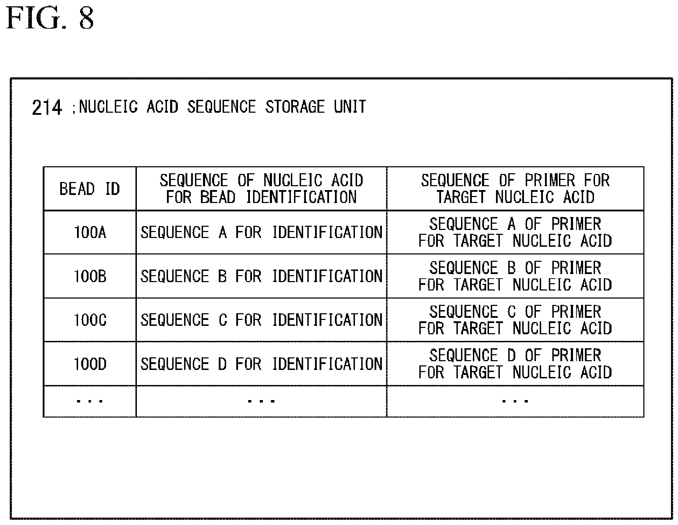

An example of a configuration of a target nucleic acid sequence identification devices 200 that realizes the method of the first embodiment described above will be described with reference to Figs. 7 and 8.

Fig. 7 is a diagram showing an example of a configuration of the target nucleic acid sequence identification device 200 in the present embodiment. The target nucleic acid sequence identification device 200 includes a substrate for beads arrangement 20 and an arithmetic device 210.

The substrate for beads arrangement 20 has a plurality of reaction vessels 21 and a plurality of sensors 30. In one reaction vessel 21, one bead 1 is arranged. On this bead 1, the above-described primer for a target nucleic acid 2 and the nucleic acid for bead identification 10 are immobilized. This nucleic acid for bead identification 10 has a nucleic acid sequence corresponding to a nucleic acid sequence of the primer for a target nucleic acid 2 immobilized on the bead 1. That is, in the reaction vessel 21, the bead 1 on which the primer for a target nucleic acid 2 and the nucleic acid for bead identification 10 having the nucleic acid sequence corresponding to the nucleic acid sequence of the primer for a target nucleic acid 2 are immobilized is arranged. A correspondence relationship between the nucleic acid sequence of the primer for a target nucleic acid 2 and the nucleic acid sequence of the nucleic acid for bead identification 10 is stored in a nucleic acid sequence storage unit 214 that the arithmetic device 210 has. A specific example of a configuration of the nucleic acid sequence storage unit 214 will be described with reference to Fig. 8.

Fig. 8 is a diagram showing an example of the configuration of the nucleic acid sequence storage unit 214 in the present embodiment. The nucleic acid sequence storage unit 214 stores bead IDs, information indicating the sequences of the nucleic acid for bead identification 10, and information indicating the sequences of the primer for a target nucleic acid 2, in a state of being correlated with each other. The bead IDs are items of information for identifying the type of the bead 1, that is, the type of the sequence of the primer for a target nucleic acid 2 immobilized on the bead 1. More specifically, a bead ID-100A correlates with a sequence A for identification and a sequence A of primer for a target nucleic acid. That is, according to the information stored in the nucleic acid sequence storage unit 214, if the nucleic acid sequence of the nucleic acid for bead identification 10 is identified, the sequence of the primer for a target nucleic acid 2 which corresponds to this nucleic acid sequence of the nucleic acid for bead identification 10 can be identified.

Returning to Fig. 7, the arithmetic device 210 has, for example, a central processing unit (CPU), and has a nucleic acid elongation determination unit 211, a bead identification unit 212, and a target nucleic acid sequence identification unit 213 as functional units thereof.

The nucleic acid elongation determination unit 211 determines the occurrence or non-occurrence of the nucleic acid elongation in the reaction vessel 21 for each reaction vessel 21 based on the amount of protons generated in each reaction vessel 21 which is detected by the sensor 30. Specifically, the nucleic acid elongation determination unit 211 has, in advance, information indicating the correspondence between an output current value of the sensor 30 and the amount of protons generated. The nucleic acid elongation determination unit 211 obtains the output current value of the sensor 30, and based on the obtained output current value, estimates the amount of protons generated. The nucleic acid elongation determination unit 211 determines the occurrence or non-occurrence of the nucleic acid elongation based on the estimated amount of protons generated. The sensor 30 is provided for each reaction vessel 21. The nucleic acid elongation determination unit 211 obtains the output current value of the sensor 30 in each reaction vessel 21, thereby determining the occurrence or non-occurrence of the nucleic acid elongation for each reaction vessel 21.

The bead identification unit 212 identifies the sequence of the nucleic acid for bead identification 10 for each reaction vessel 21 based on the occurrence or non-occurrence of the nucleic acid elongation determined by the nucleic acid elongation determination unit 211. The specific procedure for identifying the sequence of the nucleic acid for bead identification 10 has been described in detail in the above section (Identification of nucleic acid for bead identification), and therefore the explanation thereof will be omitted.

The target nucleic acid sequence identification unit 213 identifies the sequence of the primer for a target nucleic acid 2 for each reaction vessel 21 based on the sequence of the nucleic acid for bead identification 10 identified by the bead identification unit 212. Specifically, the target nucleic acid sequence identification unit 213 acquires information indicating the sequences of the nucleic acid for bead identification 10 identified by the bead identification unit 212. The target nucleic acid sequence identification unit 213 searches the nucleic acid sequence storage unit 214 using the acquired information indicating the sequences of the nucleic acid for bead identification 10 as a search key. As described above, the nucleic acid sequence storage unit 214 stores the information indicating the sequences of the nucleic acid for bead identification 10, and the information indicating the sequences of the primer for a target nucleic acid 2, in a state of being correlated with each other. Therefore, as a result of the search in the nucleic acid sequence storage unit 214 using the information indicating the sequences of the nucleic acid for bead identification 10 as the search key, the target nucleic acid sequence identification unit 213 obtains the information indicating the sequence of the primer for a target nucleic acid 2, which corresponds to the information indicating the sequence of the nucleic acid for bead identification 10. That is, the target nucleic acid sequence identification unit 213 specifies the sequence of the primer for a target nucleic acid 2 which corresponds to the sequence of the nucleic acid for bead identification 10. The target nucleic acid sequence identification unit 213 identifies the sequence of the primer for a target nucleic acid 2 which corresponds to the sequence of the nucleic acid for bead identification 10 for each reaction vessel 21 by repeating the above-described procedure for each reaction vessel 21.

According to the target nucleic acid sequence identification device 200 of the present embodiment, even if it is not identified that which type of the bead 1 is arranged in which reaction vessel 21, the primer for a target nucleic acid 2 in which the nucleic acid elongation reaction has occurred can be identified. That is, according to the target nucleic acid sequence identification device 200 of the present embodiment, even if the correspondence relationship between the type of the bead 1 and the number of the reaction vessel 21 is not identified, the target nucleic acid sequence contained in the sample can be identified.

The target nucleic acid sequence identification device 200 of the present embodiment can be used for detection and identification of pathogenic genes responsible for infectious diseases, detection and identification of genes related to disease such as cancers, expression analysis of disease-related genes, and the like, and is useful for diagnosis of diseases such as infectious diseases and cancers, and monitoring of therapeutic effects.

The various processes described above may be performed by recording a program for executing each process of the target nucleic acid sequence identification device 200 in the embodiment of the present invention in a computer-readable recording medium, and allowing a computer system to read and execute the program recorded in the recording medium.

The term "computer system" referred to herein may be a system including hardware such as an OS and peripheral devices. In a case of using the WWW system, the "computer system" is a system including a website providing environment (or display environment). The "computer-readable recording medium" refers to a storage device such as a writable non-volatile memory such as a flexible disk, a magneto-optical disk, a ROM, and a flash memory, a portable medium such as a CD-ROM, and a hard disk built in a computer system.

The "computer-readable recording medium" includes a medium in which a program is kept for a certain period of time, such as a volatile memory (for example, a dynamic random access memory (DRAM)) within a computer system serving as a server or a client in a case where a program is transmitted via a network such as the Internet or a communication line such as a telephone line. The program may be transmitted from a computer system in which the program is stored in a storage device or the like to another computer system via a transmission medium or by a transmission wave in a transmission medium. The "transmission medium" for transmitting a program refers to a medium having a function of transmitting information, such as a network (communication network) such as the Internet and a communication line (communication line) such as a telephone line. The above-described program may be a program for realizing some of the above-described functions. The above-described program may be a so-called difference file (differential program) which can realize the above-described function by a combination with a program already recorded in a computer system.

### [Configuration example of target protein identification device]

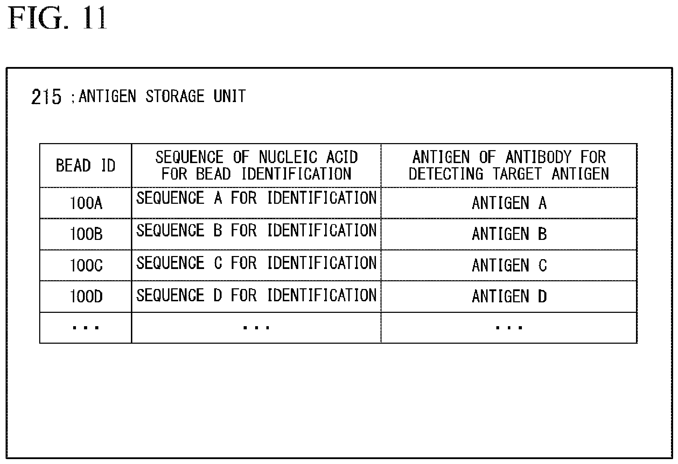

A configuration of a target protein identification device realizing the above-described method of the second embodiment can be set as a configuration including a target protein identification unit in place of the target nucleic acid sequence identification unit 213, and an antigen storage unit in place of the nucleic acid sequence storage unit 214 in the target nucleic acid sequence identification device 200 described above. Fig. 11 is a diagram showing an example of a configuration of the antigen storage unit 215 in the present embodiment. The antigen storage unit 215 stores bead IDs, information indicating the sequences of the nucleic acid for bead identification 10, and information indicating the antigens of the antibody for detecting a target antigen 5, in a state of being correlated with each other.

According to the target protein identification device of the present embodiment, even if it is not identified that which type of the bead 1 is arranged in which reaction vessel 21, the antibody for detecting a target antigen 5 which is arranged in the reaction vessel 21 in which the nucleic acid elongation reaction has occurred can be identified. That is, according to the target protein identification device of the present embodiment, even if the correspondence relationship between the type of the bead 1 and the number of the reaction vessel 21 is not identified, the target protein contained in the sample can be identified.

A target protein sequence detection device of the present embodiment can be used for detection and identification of pathogenic antigens responsible for infectious diseases, detection and identification of genes related to disease such as cancers, expression analysis of disease-related genes, and the like, and is useful for diagnosis of diseases such as infectious diseases and cancers, and monitoring of therapeutic effects.

Hereinbefore, the embodiments of the present invention have been described in detail with reference to the drawings, but the specific configuration is not limited to these embodiments, and designs and the like within the scope not departing from the gist of the present invention are also included.

### [Reference Signs List]

1 Bead
2 Primer for target nucleic acid
3 Target nucleic acid
5 Antibody for detecting target antigen
10 Nucleic acid for bead identification
11 Primer annealing region for bead identification
12 Sequence region for bead identification
20 Substrate for beads arrangement
21 Reaction vessel
30 Sensor
100, 100' Bead body
200 Target nucleic acid sequence identification device
211 Nucleic acid elongation determination unit
212 Bead identification unit
213 Target nucleic acid sequence identification unit
214 Nucleic acid sequence storage unit
215 Antigen storage unit
300 Complex probe
301 Secondary antibody
310 Nucleic acid for signal generation
311 Primer annealing region
312 Sequence for signal generation
320 Primer for signal generation
400 Target protein

## Claims

1. A method for identifying a target biological molecule, comprising:
a step (a) of bringing a sample containing biological molecules into contact with a plurality of beads on which a ligand capable of binding to one of a plurality of target biological molecules, and a nucleic acid for bead identification which has a specific sequence for each type of the ligand are immobilized;
a step (b) of arranging the plurality of beads which have been brought into contact with the sample in each of individual reaction vessels for each bead;
a step (c) of adding a reagent necessary for a nucleic acid elongation reaction to the reaction vessel in which the bead is arranged;
a step (d) of performing the nucleic acid elongation reaction in the reaction vessel in which the bead is arranged;
a step (e) of measuring an amount of protons generated in each of the reaction vessels during the nucleic acid elongation reaction;
a step (f) of determining the occurrence or non-occurrence of nucleic acid elongation in each of the reaction vessels based on the amount of protons generated; and
a step (g) of performing a nucleic acid elongation reaction in the reaction vessel in which the bead is arranged using the nucleic acid for bead identification as a template,
identifying the sequence of the nucleic acid for bead identification for each reaction vessel based on the amount of protons generated in each reaction vessel during the nucleic acid elongation reaction using the nucleic acid for bead identification as a template, and
identifying the type of the ligand for each reaction vessel based on the sequence of the nucleic acid for bead identification identified for each reaction vessel.

2. The method for identifying a target biological molecule according to claim 1, wherein the target biological molecule is a nucleic acid, and the ligand is a primer capable of being annealed to the nucleic acid that is the target biological molecule.

3. The method for identifying a target biological molecule according to claim 1, wherein the target biological molecule is a protein, and the ligand is an antibody capable of binding to the protein that is the target biological molecule.

4. The method for identifying a target biological molecule according to any one of claims 1 to 3, wherein the nucleic acid for bead identification includes a primer annealing region for bead identification and a sequence region for bead identification.

5. The method for identifying a target biological molecule according to claim 4, wherein the sequence region for bead identification includes a contiguous sequence of adenine (A), a contiguous sequence of guanine (G), a contiguous sequence of cytosine (C), or a contiguous sequence of thymine (T), or a combination of these contiguous sequences.

6. The method for identifying a target biological molecule according to any one of claims 1 to 5, wherein the nucleic acid elongation reaction in the step (d) is performed by a PCR method or an isothermal amplification method.

7. The method for identifying a target biological molecule according to any one of claims 1 to 6, wherein the measurement of the amount of protons generated in the step (e) is performed by an ISFET.

8. A bead for identifying a target biological molecule, on which a ligand capable of binding to a target biological molecule, and a nucleic acid for bead identification which has a specific sequence for each type of the ligand are immobilized.

9. A set of beads for identifying a target biological molecule, comprising:
a plurality of beads for identifying a target biological molecule, on which a ligand capable of binding to the target biological molecule, and a nucleic acid for bead identification which has a specific sequence for each type of the ligand are immobilized.

10. A target biological molecule identification device comprising:
a reaction vessel in which each bead of a plurality of beads on which a ligand capable of binding to one of a plurality of target biological molecules, and a nucleic acid for bead identification which has a specific sequence for each type of the ligand are immobilized, is to be arranged;
a detection unit which detects protons generated by a nucleic acid elongation reaction in the reaction vessel in which the bead has been arranged, for each reaction vessel;
a nucleic acid elongation determination unit which determines the occurrence or non-occurrence of nucleic acid elongation in the reaction vessel for each reaction vessel based on an amount of protons generated in the reaction vessel, which has been detected by the detection unit;
a bead identification unit that identifies the sequence of the nucleic acid for bead identification for each reaction vessel based on the occurrence or non-occurrence of the nucleic acid elongation determined by the nucleic acid elongation determination unit regarding the nucleic acid elongation reaction using the nucleic acid for bead identification as a template; and
a target biological molecule identification unit that identifies the type of the ligand for each reaction vessel based on the sequence of the nucleic acid for bead identification identified by the bead identification unit.
